# EUROPEAN PATENT APPLICATION

(11) **EP 4 295 823 A1**
(43) Date of publication of application: **27.12.2023**
(21) Application number: 22382591.0
(22) Date of filing: 22.06.2022
(51) Int. Cl.: A61F 5/44, A61F 5/445

(54) **ANTI-REFLUX OSTOMY DEVICE**

(71) Applicant: Ownmed Innovation S.L., 28009 Madrid (ES); Fundación para la Investigación Biomédica del Hospital Gregorio Marañón (FIBHGM), 28007 Madrid (ES); Fundación Instituto de Investigación Sanitaria Fundación Jiménez Díaz, 28040 Madrid (ES); Universidad Carlos III de Madrid (UC3M), 28919 Leganés (Madrid) (ES)
(72) Inventor: YE ZHANG, Ana, 28009 Madrid (ES); DESCO MENÉNDEZ, Manuel, 28007 Madrid (ES); GARCÍA-FONCILLAS LÓPEZ, Jesús, 28040 Madrid (ES)
(74) Representative: Hoffmann Eitle

(57) **Abstract**

The present invention refers to a stoma plug suitable for controlling the output of biological fluids from a colon, ileum and/or ureter (50) through a stoma (45) created by a colostomy, ileostomy or urostomy, respectively. The stoma plug comprises: a tube (10) comprising an opening (12) in a proximal portion and at least one opening (14) in a distal portion, a two-way valve system (20) comprising at least one opening (21) and a plug (24) configured to close the at least one opening (21), the two-way valve system (20) connected to the tube (10) through its proximal portion, and a cap (30) configured to cover the two-way valve system (20) and the stoma (45), comprising an opening (32) to directly access the two-way valve system (20) from the outside. The tube (10) is configured to be inserted into the colon, ileum and/or ureter (50) through its distal portion, and the two-way valve system (20) is configured to passively remain closed and to be opened when the plug (24) is displaced from the at least one opening (21).

## Description

### Technical field of the invention

The present invention pertains to the field of ostomy devices, particularly to a stoma plug for controlling the output of biological fluids through a stoma created by a colostomy, ileostomy or urostomy and to a system comprising such stoma plug and an element for managing the outputted biological fluids.

### Background of the invention

An ostomy is a surgically created opening that connects the gastrointestinal or urinary system to the skin. It allows bodily waste to pass through a surgically stoma to the exterior.

A colostomy or an ileostomy may be done as part of treatment for: cancer of the colon, rectum or anus, an inflammatory bowel disease, such as ulcerative colitis or Crohn's disease, Familial Adenomatous Polyposis (FAP), a bowel obstruction, an injury that damages the intestine, diverticulitis or birth defects of the intestines.

Colorectal cancer is the main reason of this type of procedures accounting for the 80% of all ostomies performed in Spain. It is estimated that 1.5 out of every 1000 Spanish citizens has an ostomy. This number equates to 70,000 people, with over 13,000 new cases every year. According to the International Agency for Research in Cancer (IARC), colorectal cancer is the fourth most prevalent cancer in the worldwide with and incidence in 2020 of 19.5%. According to NCBI, colorectal cancer is the fourth most common cancer and the second main cause of cancer-related deaths in the US. The risk of colorectal cancer increases with age as it is commonly diagnosed in people aged from 65 to 74. Moreover, the prevalence of colorectal cancer is increasing in Asian countries such as India, China, and Japan. As per estimates by the American Cancer Society, in 2020, the number of new cases of colon and rectal cancer in the U.S. were 104,610 and 43,340, respectively.

As per Crohn's Colitis Foundation 2019 report, approximately 1.6 million Americans currently have Inflammatory Bowel Diseases (IBD) and 70,000 new cases of IBD are diagnosed each year in the U.S. Moreover, according to the WHO report 2016, the global prevalence of colostomies, ileostomies, and urostomies was 43.0%, 41.0%, and 18.0% respectively. According to NCBI, IBD comprises ulcerative colitis or Crohn's disease, and its occurrence in European countries is gradually rising. In Europe, around 731,000 people are living with an ostomy and about 0.3% of the total population suffers from IBD. Moreover, as per the Colostomy Association Ltd., trading as Colostomy U.K., 1 in every 500 people in the U.K. is an ostomate.

However, some of the side effects of a colostomy or ileostomy include pain, bleeding, infection, blood clots, a hernia near the stoma, the stoma falling into the abdomen, a prolapse of the intestine, damage to nearby organs, dehydration and a bowel obstruction. Also, diet managing needs to be considered to help manage the following post-surgery problems: gas caused by certain foods and drinks, odor limiting foods, blocked or irritated stoma. The colon or ileum may become narrow near the stoma. certain foods may get stuck in this narrowed part and cause an obstruction (blockage), or also make the stoma swollen and irritated.

All ostomy surgeries (colostomies, ileostomies and urostomies) require life-long utility of a pouching system (i.e., stoma appliances) to store body waste (e.g., urine and/or feces). This fact derives in several implications among those people who need to use for their rest of their life these kinds of systems, which may include physical, psychological and economical functional effects such as: changes in body image, urinary/feces leakage, odor, sexual dysfunction, poor body image, fatigue, appetite loss, emotional distress related to stoma and care (i.e., anxiety, depression and worries), psychological preparation and support, sexual disfunction, complications and infections and economic loss.

Currently, pouches come in many styles and sizes, they all have a collection pouch to collect stool drainage that comes out of the stroma and an adhesive barrier (called a flange, skin barrier, or wafer) that protects the surrounding skin [8]. Pouches are made from odor-resistant materials and vary in cost. They can be either clear or opaque and come in different lengths. There are two main types of systems available:
1. One-piece pouches have both a pouch and skin barrier attached together in the same unit. When the pouch is removed, the barrier also comes off.
2. Two-piece systems are made up of a skin barrier separate from a pouch. When the pouch is taken off, the barrier stays in place.

The pouching systems can be opened at the bottom of easy emptying or closed and are taken off when they are full, allowing the adhesive skin barrier to stay on the body while the pouch may be taken off, washed out and reused.

When the colostomy puts out stool at regular (expected times), it may be able to use a stoma cover instead of always wearing a pouch. Plastic, ready-made stoma caps are also available. However, for ileostomies and urostomies, where the stool is more liquid, the stoma caps do not provide an efficient solution that can avoid spills, specially when the pouch is to be attached.

There is therefore a need for an ostomy device that can provide self-control for stool continence, no need to change the anti-reflux cap every time the stool is removed (reusable), increased self- esteem for carriers, at a low cost and with a painless insertion of the device.

### Summary of the invention

A first aspect of the invention refers to a stoma plug suitable for controlling the output of biological fluids from a colon, ileum and/or ureter (50) through a stoma (45) created by a colostomy, ileostomy or urostomy respectively. The stoma plug comprises: a tube (10) comprising an opening (12) in a proximal portion and at least one opening (14) in a distal portion, a two-way valve system (20) comprising at least one opening (21) and a plug (24) configured to close the at least one opening (21), the two-way valve system (20) connected to the tube (10) through its proximal portion, and a cap (30) configured to cover the two-way valve system (20) and the stoma (45), the cap (30) comprising an opening (32) to directly access the two-way valve system (20) from the outside. The tube (10) is configured to be inserted into the colon, ileum and/or ureter (45) through its distal portion, and the two-way valve system (20) is configured to passively remain closed and to be opened when the plug (24) is displaced from the at least one opening (21).

In a preferred embodiment, the two-way valve system (20) further comprises: a base element (22) comprising the at least one opening (21) of the two-way valve system (20), and an actuator (26). The plug (24) is configured to cover the base element (22) and seal the at least one opening (21) when the actuator (26) is not actuated. The actuator (26) is configured to displace the plug (24) and unseal the at least one opening (21) of the two-way valve system (20) when actuated and the plug (24) is further configured to reseal the at least one opening (21) of the two-way valve system (20) when the actuator (26) stops being actuated.

In a more preferred embodiment, the base element (22) is configured to perforate the plug (24) when the plug (24) is displaced by the actuator (26). In an even more preferred embodiment, the base element (22) has a spiky shape, more preferably wherein the spiky shape is essentially of a cone shape.

In an alternative preferred embodiment, the two-way valve system (20) further comprises: a spring (27), a base element (22) configured to be slidably receivable by the spring (27), and a plug stopper (29) comprising the at least one opening (21) of the two-way valve system (20). The spring (27) is configured to press the plug (24) against the plug stopper (29) and close the at least one opening (21) of the two-way valve system (20) and is further configured to longitudinally displace from the plug stopper (29) when a force is applied, thus displacing the plug (24) from the at least one opening (21) and therefore opening the at least one opening (21) of the two-way valve system (20).

In a preferred embodiment, the plug (24) comprises two pieces (24A, 24B). The two pieces (24A, 24B) are configured to remain in contact when the spring (27) presses them against the plug stopper (29) and to separate apart when the spring (27) is longitudinally displaced thus generating an aperture between the two pieces (24A, 24B) and allowing the at least one opening (21) of the two-way valve system (20) to be accessible through the aperture generated between the two pieces (24A, 24B).

In a preferred embodiment of any of the previous embodiments, the at least one opening (14) on the distal portion of the tube (10) is at least laterally placed on the tube (10).

In another preferred embodiment of any of the previous embodiments, the at least one opening (14) on the distal portion of the tube (10) is smaller than 15 mm.

In another preferred embodiment of any of the previous embodiments, wherein the tube (10) comprises a rounded tip (15) on its distal portion, configured to facilitate the insertion of the tube (10) through the stoma (45).

In another preferred embodiment of any of the previous embodiments, the tube (10) external surface further comprises at least a spiral portion configured to increase the contact surface of the tube (10) with the stoma (50).

In another preferred embodiment of any of the previous embodiments, the tube (10) is comprised of any of the materials selected from: medical grade silicone, surgical plastic or thermoplastic elastomers.

In another preferred embodiment of any of the previous embodiments, the tube (10) further comprises a lubricated layer on its external surface, configured to reduce the friction with the stoma (45) during its insertion.

In another preferred embodiment of any of the previous embodiments, the two-way valve system (20) further comprises a heat-sensitive hydrocolloid surface configured to stick to the external stoma mucosa (45). In a further preferred embodiment, the heat-sensitive hydrocolloid surface comprises a dressing configured to adhere to the subject's skin (40) extending radially from the two-way valve system (20). In an even further preferred embodiment, the dressing is transparent.

A second aspect of the invention refers to a stoma plug system. The stoma plug system comprises a stoma plug according to any of the previous claims, and a collecting bag or a vacuum device. The collecting bag or vacuum device is configured to connect to the two-way valve system (20) and to open the at least one opening (21) by displacing the plug (24) from the at least one opening (21). In a preferred embodiment, the collecting bag or vacuum device opens the at least one opening (21) through its connection to the two-way valve system (20). In another preferred embodiment, the vacuum device is a syringe.

### Brief description of the drawings

To enable better understanding of the present disclosure, and to show how the same may be carried into effect, reference will now be made, by way of example only, to the accompanying schematic drawings, in which:
Fig. 1 shows an exploded view of a stoma plug according to at least one of the embodiments of the invention.
Fig. 2 shows a perspective view of a stoma plug according to at least one of the embodiments of the invention.
Fig. 3 shows a schematic cross-sectional view of a stoma plug according to at least one of the embodiments of the invention.
Fig. 4 shows a schematic cross-sectional view of an actuated stoma plug according to at least one of the embodiments of the invention.
Fig. 5 shows another schematic cross-sectional view of a stoma plug according to at least one of the embodiments of the invention.

### Description of the invention

### Definitions

The term "two-way valve system" is preferably understood as a valve system capable of allowing fluids to flow in both senses. The fluid flow sense will be determined by the pressures at each side of the valve, such that the flow will go from the side where the fluid pressure is higher to the side where the fluid is lower.

The term "vacuum device" is preferably understood as any device capable of creating a negative pressure. It may therefore be a vacuum connection from a hospital, a portable fluid vacuum device or simpler device such as a syringe.

### Description

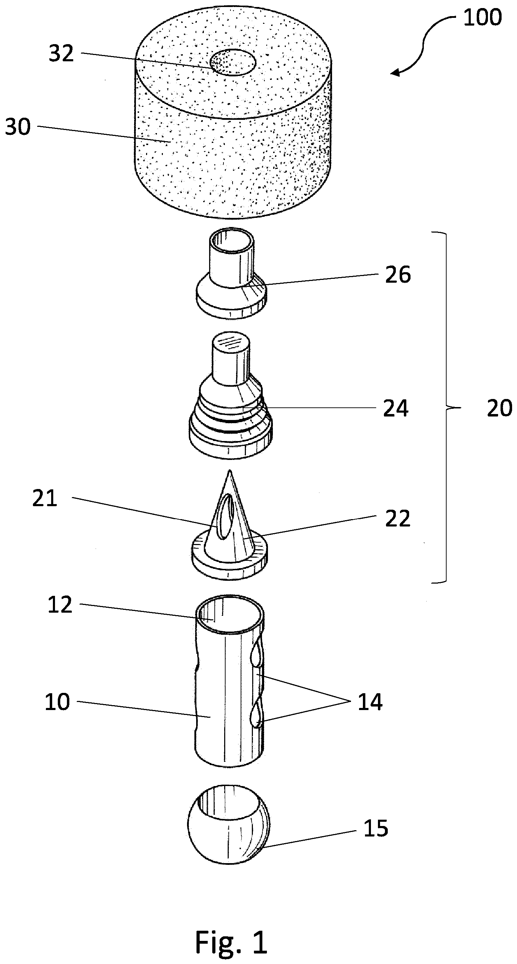

A first aspect of the present invention refers to a stoma plug suitable for controlling the output of biological fluids from a colon, ileum and/or ureter (50) through a stoma (45) created by a colostomy, ileostomy or urostomy. As shown with respect to Fig. 1, the stoma plug 100 comprises: a tube 10 comprising an opening 12 in a proximal portion and at least one opening 14 in a distal portion. It is noted that in Fig. 1 the tube 10 comprises two openings 14 in the distal portion but in other embodiments the tube may comprise only one opening 14 or more than two openings 14 in the distal portion. The stoma plug 100 further comprises a two-way valve system 20 comprising at least one opening 21 and a plug 24 configured to close the at least one opening 21. The two-way valve system 20 connected to the tube 10 through its proximal portion. It is noted that in Fig. 1 the two-way valve system 20 comprises other optional elements 22 and 26 but in other embodiments the plug may comprise only at least one opening 21 and a plug 24 configured to close the at least one opening 21. Lastly, the stoma plug 100 comprises a cap 30 configured to cover the two-way valve system 20 and the stoma 45. The cap 30 comprises an opening 32 to directly access the covered two-way valve system 20 from the outside.

The tube 10 is configured to be inserted into the colon, ileum and/or ureter 50 through its distal portion, and the two-way valve system 20 is configured to passively remain closed and to be opened when the plug 24 is displaced from the at least one opening 21.

Having a tube comprising an opening 12 in a proximal portion and at least one opening 14 in a distal portion advantageously allows it to fluidly connect the colon, ileum and/or ureter 50 biological fluids with the two-way valve system 20 through the at least one opening 14 of the distal portion. In use the tube 10 is inserted into the colon, ileum and/or ureter 50 through its distal portion and the two-way valve system 20 is connected to the tube 10 through its proximal portion. Therefore, the biological fluids present at the colon, ileum and/or ureter 50 can pass through the opening 14 of the tube 10 and reach the opening 12 where the two-way valve system 20 is connected. Therefore, the two-way valve system 20 is configured to control the pass of the biological fluids through the stoma 45.

The two-way valve system 20 advantageously allows it to provide a way to control the flow of fluids in both directions, the flow of the biological fluids from the colon, ileum and/or ureter 50 through the tube 10 towards the exterior, and the flow of any fluid from the exterior to the colon, ileum and/or ureter 50 through the tube 10. Therefore, it allows to control the passage of any fluids at the stoma level. It is noted that in Fig. 1 the two-way valve system 20 further comprises elements 22 and 26 but in other embodiments the same advantages can be achieved using other elements.

It is noted that the person skilled in the art may devise several ways of making a two-way valve system 20. Although the present invention mainly describes two alternative ways of such a two-way valve system 20, it is not limited to any particular type of two-way valve system 20 comprising at least one opening 21 and a plug 24 configured to close that plug passively and to be opened when the plug 24 is displaced form the at least one opening 21. Also, more complex systems comprising more than one plug 24 and not being configured to be passively closed but actively closed (either manually or mechanically) may be envisaged by the person skilled in the art.

The cap 30 advantageously covers both the two-way valve system 20 and the stoma external mucosa 45, so that external elements such as clothes do not interact with neither the two-way valve system 20 which could lead to an undesired opening of the valve nor the stoma external mucosa 45, which could lead to a pain source. The cap 30 comprises an opening 32 to access the two-way valve system 20 so that the fluids can be extracted and optionally to operate the valve, i.e. to open it when desired. It is noted that in although in Fig. 1 the opening 32 is centred in the cap surface, the opening 32 may be located in any part of the cap 30 surface. The cap may comprise different shapes in order to provide a slimmer and less noticeable device. It may also comprise different materials, for example elastic and/or flexible materials that can adapt to the user's skin surface 40 and/or the stoma external mucosa 45.

It is also noted that the tube 10, the two-way valve system 20 and the cap 30 shapes and designs of Fig. 1 are only for illustrative purposes. Therefore, the tube 10 may have a section different to a circular one, or may be longer or shorter. The two-way valve system 20 may be more or less compact and have different ways of functioning. Also, the cap 30 may take different shapes other than cylindrical to cover the two-way valve system 20 and the stoma 45. Similarly, the different openings 12, 14, 21, 32 may take different shapes and the circular and oval ones in the drawings are for illustrative purposes. Therefore, different opening shapes and patterns may be used as the person skilled in the art will notice. For example, they may be shaped in form of an oval or ellipse, or when more than one opening of one type is present, they may be disposed in different configurations as to maximise the flow rate capacity and/or to reduce the fluid turbulence when a fluid flows through them.

Fig. 1 comprises an optional rounded tip 15 at the distal portion of the tube 10 that it may not be present in other embodiments. Also, the shape of such tip may have a different shape.

The two-way valve system (20) is configured to passively remain closed. Advantageously, this allows for the stoma plug to be configured to prevent the flow of the biological fluids when in use. Therefore, the stoma plug according to any embodiment of the first aspect of the invention provides an ostomy device that can provide self-control for stool continence as only when the plug 24 is removed from the opening 21 the biological fluids such as the stool can flow outwards from the colon, ileum and/or ureter 50 through the stoma 45. Moreover, as the two-way valve system 20 can be closed again once opened, there is no need to change the anti-reflux cap every time the stool is removed, making it reusable. Altogether, this provides an increased self-esteem for carriers, at a low cost. Moreover, as the tube 10 is configured to be inserted into the stoma, it provides all these advantages with a painless insertion of the device.

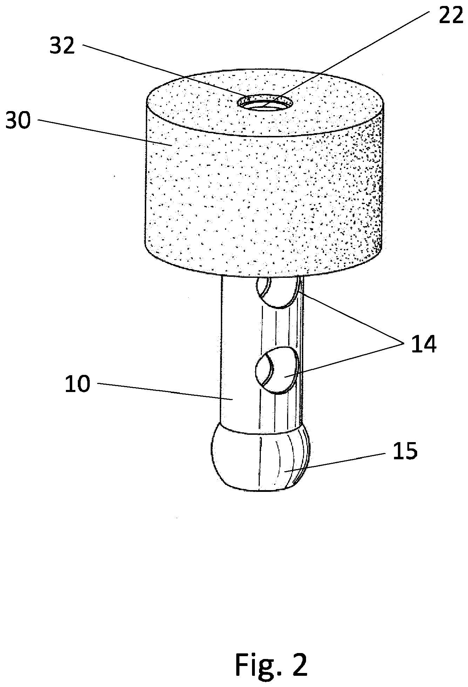

Fig. 2 shows a perspective view of a stoma plug according to at least one of the embodiments of the invention. It comprises a tube 10, a two-way valve system 20 (not seen) and a cap 30. It can be seen that the cap 30 covers the two-way valve system 20 and that the opening 32 of the cap 30 allows to directly access the two-way valve system 20 from the outside. It is noted that the tube 10 comprises more than one opening 14 in the distal portion, but these are optional and in other embodiments, the tube 10 may comprise only one or a different number of openings 14 in the distal portion of the tube 10. Moreover, the stoma plug of Fig. 2 further comprises an optional rounded tip 15 in the distal portion of the cap 10 and other embodiments may not comprise such rounded tip 15.

In a preferred embodiment, as shown in Fig 1, the two-way valve system 20 further comprises: a base element 22 comprising the at least one opening 21 of the two-way valve system 20, and an actuator 26. The plug 24, is configured to cover the base element 22 and seal the at least one opening 21 when the actuator 26 is not actuated. Moreover, the actuator 26 is configured to displace the plug 24 and unseal the at least one opening 21 of the two-way valve system 20 when actuated. The actuator 26 may be actuated in different ways. For example, as shown in Fig.1 the actuator 26 can longitudinally displace the plug 24 towards the base element 22, and allow the at least one opening 21 to be unsealed. However, the person skilled in the art may appreciate there are many ways an actuator can displace a plug 24 from an opening 21.Moreover, in this preferred embodiment, the plug 24 is further configured to reseal the at least one opening 21 of the two-way valve system 20 when the actuator 26 stops being actuated. Therefore, after the actuator 26 is actuated, the at least one opening 21 is resealed by the plug 24. For example, in the embodiment of Fig. 1, when the actuator 26 stops being actuated the plug 24 is shaped in a spring-like pattern that when made of an elastic material, after being compressed during the displacement by the actuator 26, recovers its original position, therefore resealing the at least one opening 21 of the two-way valve system 20. Advantageously, this configures the two-way valve 20 to be configured to passively remain closed whenever the actuator 26 is not actuator. This means that the stoma plug is configured to be closed and sealed whenever the valve it's not actuated, so it further prevents the flow of the biological fluids when in use, i.e. in the daily life of the user. and provides self-control for stool continence. Moreover, as the two-way valve system 20 can be closed again once opened, there is no need to change the anti-reflux cap every time the stool is removed, making it reusable. The plug 24 may comprise an elastic and or flexible material that can recover its original shape once an external force quits being applied into it. Therefore, the plug 24 may act as a spring element.

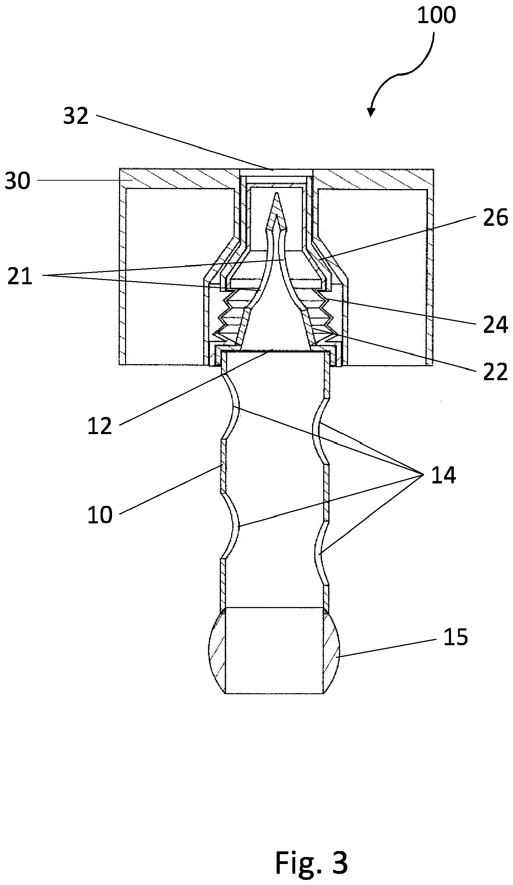

More preferably, the base element 22 is configured to perforate the plug 24 when the plug 24 is displaced by the actuator 26. Fig. 3 shows a schematic cross-sectional view of a stoma plug 100 according to at least one of the embodiments of the invention. It is noted that the stoma plug 100 of Fig. 3 comprises a tube 10, a two-way valve system 20 and a cap 30. However, it further comprises the optional elements: several openings 14 in the distal portion of the tube 10, a rounded tip 15 on the distal portion of the tube 10, comprises two openings 21. However, in other embodiments, there may be one or different number of openings 14 in the distal portion of the tube 10, may be no rounded tip 15 on the distal portion of the tube 10 and one or more than two openings 21. It is noted that the base element 22 may perforate the plug in several ways. The person skilled in the art may devise different ways in which the base element 22 is configured to perforate the plug 24 when the plug 24 is displaced by the actuator 26. For example, the base element 22 may comprise a perforating element that is actuated by the actuator 26 when the actuator is actuated, so that when the plug 24 is displaced towards the base element 22, the perforating element is also actuated and perforates the plug 24.

It can be seen that in Fig. 3 the plug 24 is configured to seal the at least one opening 21 of the base element 22, and that the actuator 26 is configured to longitudinally displace the plug 24 towards the base element 22 to unseal the at least one opening 21. However, it can be noticed that when the plug 24 is displaced by the actuator 26, it does not allow to unseal the element unless the base element 22 perforates the plug 24, therefore generating an aperture from wherein the fluids can flow.

Advantageously this configures the plug 24 to be pre-sealed before the first use, therefore providing an additional security measure against biological fluids from the stoma to leak to the outside when in use until the first aperture of the two-way valve system through the actuation of the actuator. Once the plug 24 is perforated by the base element 22 and after the actuator 26 stops being actuated, the plug 24 reseals the opening 21, but the plug 24 remains perforated. It is noted that the opening 21 seal is not compromised by the perforation of the plug 24.

In an even more preferred embodiment, the base element 22 has a spiky shape, more preferably wherein the spiky shape is essentially of a cone shape. Advantageously, this configures the base element 22 to be able to perforate the plug 24, when the plug 24 is displaced towards the base element 22 by the actuator 26. Therefore, the plug 24 displacement by the actuator 26 is the one that causes the base element 22 to perforate the plug 24.

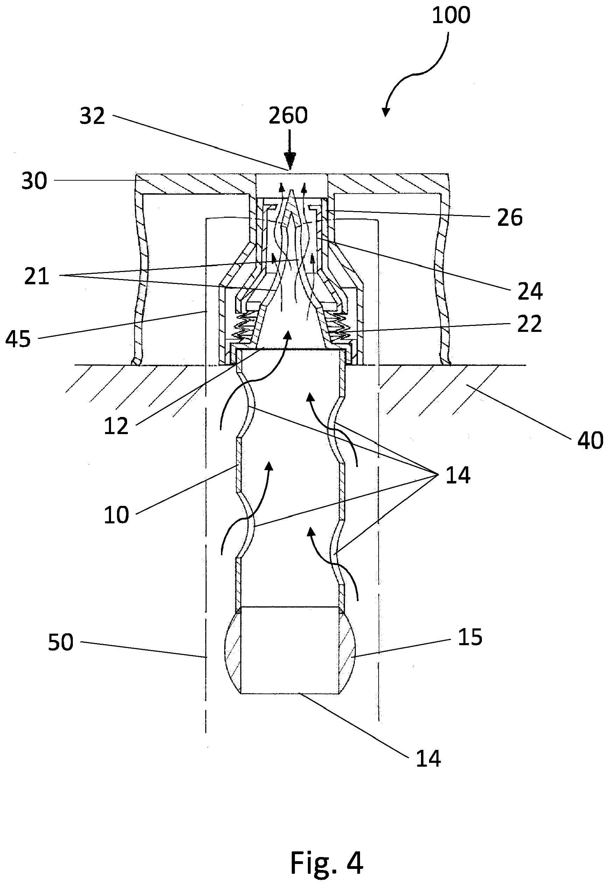

Fig. 4 shows the schematic cross-sectional view of an actuated stoma plug 100 inserted in a stoma 50, according to at least one of the embodiments of the invention. It is noted that the stoma plug 100 of Fig. 4 comprises a tube 10, a two-way valve system 20 and a cap 30. However, it further comprises the optional elements: several openings 14 in the distal portion of the tube 10, a rounded tip 15 on the distal portion of the tube 10, comprises two openings 21. However, in other embodiments, there may be one or different number of openings 14 in the distal portion of the tube 10, may be no rounded tip 15 on the distal portion of the tube 10 and one or more than two openings 21. Moreover, it's noted that the stoma plug 100 is inserted into a stoma 40 and that the cap 30 is configured to cover the two-way valve system 20 and the stoma external mucosa 45, and adapted to the skin surface 40. It is noted that the cap 30 may take several shapes to adapt to the skin surface 40.

The stoma plug 100 of Fig. 4 is actuated as shown by arrow 260 through the opening 32 of the cap 30, such that the actuator 26 has displaced the plug 24 and unsealed the at least one opening 21 of base element 22. Moreover, the base element 22, has a spiky shape in form of a cone, that has perforated the plug 24. In this configuration, the two-way valve-system 20 has the plug 24 displaced from the at least one opening 21, therefore fluidly connecting the biological fluids of the colon, ileum and/or ureter 50 (such as the stool) through the openings 14 at the distal portion of the tube 10, which in turn provides access to the openings 21 of the base element 22 through the opening 12 at the proximal portion of the tube 10. Moreover, as the actuator 26 has been actuated (as shown by arrow 260), the plug 24 has unsealed the openings 21 and the biological fluids of the colon, ileum and/or ureter 50 can reach the exterior through the opening 32 of the cap 30. Therefore, when the actuator 26 is actuated, the biological fluids of the stoma 50 are fluidly connected to the exterior of the body.

It is noted that the two-way valve system 20, allows for an external fluid to be inserted into the colon, ileum and/or ureter 50 through the stoma 45 when the actuator 26 is actuated, as it is fluidly connected to the interior of the colon, ileum and/or ureter 50. For example, a mixture of lactulose and water or lactulose and cola drink may be introduced into the colon, ileum and/or ureter 50 through the stoma 45 to fight or prevent an intestinal obstruction.

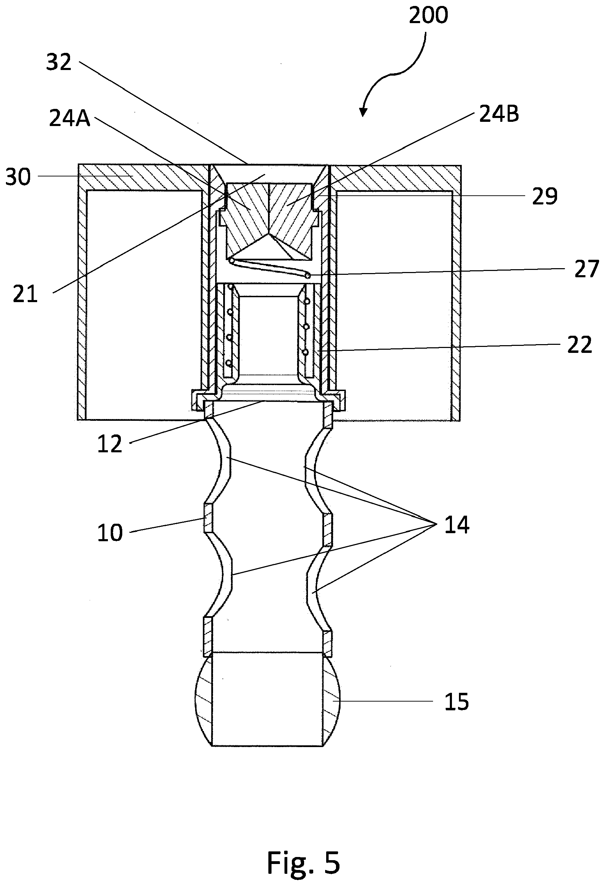

Alternatively, in another preferred embodiment, as shown in Fig. 5, the two-way valve system further comprises: a spring 27, a base element 22 configured to be slidably receivable by the spring 27, and a plug stopper 29 comprising the at least one opening 21 of the two-way valve system 20. It is noted that the stoma plug 200 of Fig. 5 comprises a tube 10, a two-way valve system 20 and a cap 30. However, it further comprises the optional elements: several openings 14 in the distal portion of the tube 10, a rounded tip 15 on the distal portion of the tube 10, and a two-piece plug 24. However, in other embodiments, there may be one or different number of openings 14 in the distal portion of the tube 10, may be no rounded tip 15 on the distal portion of the tube 10 and the plug 24 may not comprise more than one piece or comprise several pieces. In some embodiments, as in Fig. 5, the base element 22 is a fixed element of the two-way valve system, so that when the spring is compressed, it is not displaced. The base element 22 holds the spring into a particular direction so that the compression direction is constant. The base element 22 is further the element of the two-way valve system 20 through which the two-way valve system 20 is connected to the proximal portion of tube 10.

The spring 27 is configured to press the plug 24 against the plug stopper 29 and close the at least one opening 21 of the two-way valve system 20. It is noted that the person skilled in the art may envisage several ways in which a spring 27 can press a plug 24 against a plug stopper 29 to close the at least one opening 21 of the two-way valve system 20. For example, the spring 27 may be a compression spring and be at least partially compressed so that it exerts an extension force. If the plug 24 is on one side of the spring 27 and the base element 22 is on the other side as a support, when the plug 24 is oriented towards the plug stopper, the spring 27 presses the plug 24 against the plug stopper 29 and close the at least one opening 21 of the two-way valve system 20. Alternatively, the spring 27 may be an extension spring and be at least partially stretched so that it exerts a compression force. If the plug 24 is attached to one side of the spring 27 and the base element 22 is on the other side as a support, when the plug stopper 29 is between the spring 27 and the base element 22, the spring 27 presses the plug 24 against the plug stopper 29 and close the at least one opening 21 of the two-way valve system 20. Equivalent systems may be designed by the person skilled in the art using one or more torsion springs, drawbar springs, volute springs, and any other type of spring as known in the art. In Fig. 5, the spring 27 is a compression spring, that presses the plug 24 against the plug stopper 29, therefore closing the at least one opening 21.

Moreover, the spring 27 is further configured to longitudinally displace from the plug stopper 29 when a force is applied, thus displacing the plug 24 from the at least one opening 21 and therefore opening the at least one opening 21 of the two-way valve system 20. It is noted that the person skilled in the art may envisage several ways in which a spring 27 can be displaced from the plug stopper 29 when a force is applied. Several options may be defined according to the type of spring as defined above. For example, in Fig. 5 the compression spring 27 can be compressed, and as the base element 22 is fixed, the plug 24 is thus displaced from the at least one opening 21 of the two-way valve system 20, leading to the opening of the at least one opening 21.

Advantageously, having a spring-actuated two-way valve system 20 allows for a reliable opening and closing mechanism, that works in a positive feedback to reinforce the sealing of the at least one opening 21 of the two-way valve system 20. For example, in the embodiment as shown in Fig. 5, if the pressure of the biological fluids of the stoma increases, the pressure further presses the plug 24 against the plug stopper 29 and further closes the at least one opening 21 of the two-way valve system 20. By this way the stoma plug is configured to further prevent the involuntary flow of the biological fluids when in use.

In a further preferred embodiment, as shown in Fig. 5, the plug 24 comprises two pieces 24A, 24B. The two pieces 24A, 24B are configured to remain in contact when the spring 27 presses them against the plug stopper 29 and to separate apart when the spring 27 is longitudinally displaced, thus generating an aperture between the two pieces 24A, 24B and allowing the at least one opening 21 of the two-way valve system 20 to be accessible through the aperture generated between the two pieces 24A, 24B.

The two pieces 24A, 24B may be two equal pieces or may be pieces that have different shapes. Altogether, the two pieces 24A, 24B when in contact they fully close the at least one opening 21 of the two-way valve system 20. The person skilled in the art may note this can be done in several ways. For example, one of the pieces 24A may be a ring and the other one 24B be a plug closing the inner circle left by the ring of the first piece 24A. In the example of Fig. 5, the two pieces 24A, 24B are pieces each covering half of the circumference left by the at least one opening 21. Therefore, both pieces 24A, 24B have the same shape. However, in other embodiments these may have different shapes. It is noted, that in the embodiments when there are more than one opening 21, each of the two pieces 24A, 24B may close one or more than one of the opening 21.

The two pieces 24A, 24B may be configured to separate apart when the spring 27 is longitudinally displaced in several ways. For example, each of the two pieces 24A, 24B may have a lever or a ramp that interacts with the spring 27 or any of the surrounding elements such as the base element 22, so that the two pieces 24A, 24B move in opposite directions outwardly, therefore generating an aperture between the two pieces 24A, 24B. Through this aperture, the at least one opening 21 of the two-way valve system 20 can be accessed from the interior, i.e. the plug 24 is no longer closing the at least one opening 21. Consequently, as the at least one opening 21 is fluidly connected to the biological fluids in the tube distal portion through the opening 12 in the proximal portion of the tube 10 and the at least one opening 14 in the distal portion of the tube 10, the biological fluids at the distal portion of the tube 10 are fluidly communicated with the exterior.

In use, the two-way valve system 20 of the embodiment according to Fig. 5 is configured to be opened when the spring 27 is longitudinally displaced towards the base element 22, i.e. by compressing the spring. This makes the two pieces 24A, 24B to separate apart, as each have a ramp shaped base that interacts with the base element 22 and displaces them outwardly, allowing the biological fluids from the colon, ileum and/or ureter 50 to be fluidly connected to the at least one opening 21 of the two-way valve system 20 through the aperture generated between the two pieces 24A, 24B.

Advantageously, having the at least one opening 21 of the two-way valve system 20 accessible through the aperture generated between the two pieces 24A, 24B allows for a better flow through the at least one opening 21, as the aperture generated between the two pieces 24A, 24B is aligned with the at least one opening 21. Having these aligned, reduces the turbidity of the fluid flow which can reduce the probability of clogging the two-way valve system by any small solid present in the fluid.

It is noted that in this alternative preferred embodiment, the spring 27 is configured to longitudinally displace from the plug stopper 29 when a force is applied. However, this force may be applied by the user at a will or using any external element through the opening 32 of the cap 30.

In a preferred embodiment of any of the previous embodiments, the at least one opening 14 of the distal portion of the tube 10 is at least laterally placed on the tube 10. As shown in Figs. 1-5 some of the openings 14 in the tube 10 distal portion are laterally placed. However, in other embodiments, there may be an opening 14 on the tip but partially laterally placed, or when the stoma plug comprises only one opening 14 in the tube 10 distal portion, the opening may be only lateral or at least laterally placed in the tube 10, so that part of the opening is at the tip and part on the lateral of the tube 10. Advantageously, this allows for the biological fluids in the colon, ileum and/or ureter 50 to keep flowing into the tube 10 through the at least one opening 14, whenever a big piece of stool is placed at the tip of the tube 10. As the stool exercises a pressure at the colon, ileum and/or ureter 50 end due to the peristaltic movement at the point, the at least one opening 14 at the tip of the tube 10 at the distal portion may be blocked, but the at least laterally placed portion of the at least one opening 14, is kept clear from the stool, while the biological fluids can keep reaching such lateral at least one opening 14 at least laterally placed on the tube 10.

In another preferred embodiment of any of the previous embodiments, the at least one opening on the distal portion 14 of the tube 10 is smaller than 15 mm. The present stoma plug is preferably designed for the control of liquids such that of ileostomies and urostomies, as well as some colostomies. Therefore, the at least one opening 14 on the distal portion of the tube 10 may be used to control the size of the elements that reaches the two-way valve system 20. Advantageously, having the at least one opening 14 on the distal portion of the tube 10 smaller than 15 mm configures the stoma plug to prevent any element such as stool bigger than 15mm to be extracted when the two-way valve system 20 is opened, which could clog the two-way valve system 20. It is noted that in Figs. 1-5, the at least one opening 14 are circles, but that in other embodiments, these one opening may take different shapes as the person skilled in the art may foresee such as ovals, squares or any other shape. In the case of the Figs. 1-5 the size of the at least one opening 14 is determined by the diameter of the openings, while in other embodiments, the size of the at least one opening 14 is defined by the maximum diameter of the opening 14 in any direction over the surface of the tube 10. In a more preferred embodiment, the at least one opening 14 on the distal portion of the tube 10 is smaller than 10 mm.

In another preferred embodiment of any of the previous embodiments, the tube 10 comprises a rounded tip 15 on its distal portion, configured to facilitate the insertion of the tube 10 into the stoma 50. As the tube 10 is configured to be introduced into a colon, ileum and/or ureter 50, having a rounded tip 15, further facilitates the insertion of the tube through the stoma 45, such that the internal mucosa of the colon, ileum and/or ureter 50 can slide as the tube 10 is introduced through the stoma 45 into the colon, ileum and/or ureter 50. Therefore, this feature further allows for a painless insertion of the stoma plug.

In another preferred embodiment of any of the previous embodiments, the tube 10 external surface further comprises at least a spiral portion configured to increase the contact surface of the tube 10 with the colon, ileum and/or ureter 50. Having at least a spiral portion on the externals surface of the tube, generates a rougher surface to which mucosa of the colon, ileum and/or ureter 50 can attach to. Advantageously, this provides a better grip between the internal mucosa of the colon, ileum and/or ureter 50 and the tube 10, providing a more secure attachment of the stoma plug to the colon, ileum and/or ureter 50. It is noted that many spiral configurations may be envisaged. For example, the spiral may be outwardly radially extending from the external surface of the tube 10, or it may be inwardly radially extending into the external surface of the tube 10. In some embodiments, the spiral may have different degrees of turn per length of the tube 10 and may turn in one or another sense (i.e. clockwise or counterclockwise). It may also turn in one sense in one portion of the tube 10 and in another sense in another portion of the tube 10.

In another preferred embodiment of any of the previous embodiments, the tube 10 is comprised of any of the materials selected from: medical grade silicone, surgical plastic or thermoplastic elastomers. Advantageously, this configures the tube 10 to be inserted through the stoma 45 into the colon, ileum and/or ureter 50 to be biocompatible and at least partially flexible so that it can adapt to the colon, ileum and/or ureter 50 shape. This means that in use, the tube 10 can bend when the colon, ileum and/or ureter 50 bends therefore reducing the pain and increasing the self-esteem of the user.

In another preferred embodiment of any of the previous embodiments, the tube 10 further comprises a lubricated layer on its external surface, configured to reduce the friction during its insertion through the stoma 45. This layer may comprise any antifriction substance such as oily substances or water-based lubricants, and may further comprise anaesthetic components, such as tetracaine or lidocaine. Advantageously, this configures the tube 10 to be inserted through the stoma 45 without friction and therefore increasing the ease of use and reducing the pain felt by the user.

In another preferred embodiment of any of the previous embodiments, the two-way valve system 20 further comprises a heat-sensitive hydrocolloid surface configured to stick to the stoma 45. As the colon, ileum and/or ureter 50 biological fluids may be at a higher pressure inside the stoma than the atmosphere pressure, it is desired to have the stoma plug 100, firmly attached to the stoma 45. As shown for example with respect to Fig. 4 the two-way valve system 20 interacts with the stoma 45, to fix the stoma plug 100 into the colon, ileum and/or ureter 50. Therefore, having a hydrocolloid surface that is absorbent, self-adhesive, and waterproof allows for the stoma plug 100 to be firmly affixed to the stoma. Moreover, a heat-sensitive surface may only adhere to heated surfaces such as the stoma, making the stoma plug 100 easy to manipulate before the insertion into the colon, ileum and/or ureter 50. Advantageously, having the two-way valve system 20 further comprises a heat-sensitive hydrocolloid surface configured to stick to the external stoma mucosa 45 configures the stoma plug 100, 200 to be kept in place when the internal pressure in the colon, ileum and/or ureter 50 increases over the atmosphere pressure.

In a more preferred embodiment, the heat-sensitive hydrocolloid surface comprises a dressing configured to adhere to the subject's skin 40 extending radially from the two-way valve system 20. Advantageously, this further increases the adherence of the stoma plug 100, 200, by further adhering to the user's skin. It is noted that the dressing may extend radially with different lengths in each direction and that the shape may be adapted to the location of the stoma 45 and/or to the size of the stoma 45. In an even more preferred embodiment the dressing is transparent, providing a less visible design and further increasing the self-esteem of the subject.

In another preferred embodiment, the stoma plug 100, 200 further comprises a pressure sensor to monitor the pressure inside the colon, ileum and/or ureter 50. The two-way valve system 20 may then be opened automatically, when the pressure sensor detects that a certain pressure threshold is reached or it may indicate the user through a feedback element that the two-way valve system 20 shall be opened to drain the biological fluids of the colon, ileum and/or ureter 50.

A second aspect of the invention refers to a stoma plug system comprising a stoma plug 100, 200 according to any of the stoma plugs of the first aspect of the invention and a collecting bag or a vacuum device. The collecting bag or vacuum device is configured to connect to the two-way valve system 20 and to open the at least one opening 21 displacing the plug 24 from the at least one opening 21. Preferably, the collecting bag or vacuum device is configured to connect to the two-way valve system 20 through the opening 32 of the cap 30.

Therefore, when the collecting bag or the vacuum device is connected to the two-way valve system 20, the stoma plug is open, and the biological fluids of the colon, ileum and/or ureter 50 can get to the exterior through the stoma plug. Both the collecting bag and the vacuum device are thus configured to deal with the biological fluids that may come out form the stoma plug. The collecting bag can be any type of collecting bag as known in the stoma device industry. Thus, the collecting bag may be a pouch opened at the bottom so that the contents collected can be disposed through the opening on the bottom, or it may be a pouch closed at the bottom and it may therefore be replaced when the contents need to be disposed. The vacuum device therefore either comprises a collecting recipient to store the biological fluids or is connected to a recipient where these can be stored.

It is noted that while the collecting bag collects the biological fluids of the colon, ileum and/or ureter 50 that passively exits the stoma plug, the vacuum device actively extracts the biological fluids of the colon, ileum and/or ureter-50 by using a negative pressure when compared to the internal pressure of the colon, ileum and/or ureter 50.

The collecting bag or vacuum device opens the at least one opening 21 displacing the plug 24 from the at least one opening 21 in several ways. For example, it may open the at least one opening 21 by directly displacing the plug 24 from the at least one opening 21 when it is connected to the two-way valve system 20, or it may do so by actuating on an actuator 26 at the moment it is when it is connected to the two-way valve system 20. Alternatively, the collecting bag or vacuum device may comprise a second mechanism on the connection element to the at least one opening 21 of the two-way valve system 20 that may be manually or electrically actuated to open the two-way valve system 20.

In the embodiments, wherein the stoma plug 100, 200 comprises a pressure sensor to monitor the pressure inside the colon, ileum and/or ureter 50, the sensor may send a signal to the user indicating when is the adequate moment to drain the biological fluids of the colon, ileum and/or ureter 50 by using the collecting bag or a vacuum device.

Advantageously, a stoma plug system comprising a stoma plug 100, 200 according to any of the stoma plugs of the first aspect of the invention and a collecting bag or a vacuum device allows the user to either allow the biological fluids of the colon, ileum and/or ureter 50 to passively be drained by using a collecting bag, or to manually and actively draining the biological fluids of the colon, ileum and/or ureter 50 using the vacuum provided by the vacuum device.

In a preferred embodiment, the vacuum device is a syringe. Advantageously, the user can use the syringe to extract in a short period of time the biological fluids of the colon, ileum and/or ureter 50 through the stoma plug. Therefore, the user can easily release the biological fluids of the colon, ileum and/or ureter 50 through the stoma 45 in fast and clean way. The syringe may then be emptied in a toilet. The syringe may be reusable or disposable.

## Claims

1. A stoma plug suitable for controlling the output of biological fluids from a colon, ileum and/or ureter (50) through a stoma (45) created by a colostomy, ileostomy or urostomy respectively, comprising:
a) a tube (10) comprising an opening (12) in a proximal portion and at least one opening (14) in a distal portion,
b) a two-way valve system (20) comprising at least one opening (21) and a plug (24) configured to close the at least one opening (21), the two-way valve system (20) connected to the tube (10) through its proximal portion, and
c) a cap (30) configured to cover the two-way valve system (20) and the stoma (45), comprising an opening (32) to directly access the two-way valve system (20) from the outside,
wherein the tube (10) is configured to be inserted into the colon, ileum and/or ureter (50) through its distal portion, and
wherein the two-way valve system (20) is configured to passively remain closed and to be opened when the plug (24) is displaced from the at least one opening (21).

2. The stoma plug according to the previous claim, wherein the two-way valve system (20) further comprises:
a base element (22) comprising the at least one opening (21) of the two-way valve system (20), and
an actuator (26),
wherein the plug (24), is configured to cover the base element (22) and seal the at least one opening (21) when the actuator (26) is not actuated,
wherein the actuator (26) is configured to displace the plug (24) and unseal the at least one opening (21) of the two-way valve system (20) when actuated and wherein the plug (24) is further configured to reseal the at least one opening (21) of the two-way valve system (20) when the actuator (26) stops being actuated.

3. The stoma plug according to the previous claim, wherein the base element (22) is configured to perforate the plug (24) when the plug (24) is displaced by the actuator (26).

4. The stoma plug according to claims 2 or 3, wherein the base element (22) has a spiky shape, more preferably wherein the spiky shape is essentially of a cone shape.

5. The stoma plug according to claim 1, wherein the two-way valve system (20) further comprises:
a spring (27),
a base element (22) configured to be slidably receivable by the spring (27), and
a plug stopper (29) comprising the at least one opening (21) of the two-way valve system (20), wherein the spring (27) is configured to press the plug (24) against the plug stopper (29) and close the at least one opening (21) of the two-way valve system (20) and
wherein the spring (27) is further configured to longitudinally displace from the plug stopper (29) when a force is applied, thus displacing the plug (24) from the at least one opening (21) and therefore opening the at least one opening (21) of the two-way valve system (20).

6. The stoma plug according to the previous claim, wherein the plug (24) comprises two pieces (24A, 24B), and wherein the two pieces (24A, 24B) are configured to remain in contact when the spring (27) presses them against the plug stopper (29) and to separate apart when the spring (27) is longitudinally displaced, thus generating an aperture between the two pieces (24A, 24B) and allowing the at least one opening (21) of the two-way valve system (20) to be accessible through the aperture generated between the two pieces (24A, 24B).

7. The stoma plug according to any of the previous claims, wherein the at least one opening (14) on the distal portion of the tube (10) is smaller than 15 mm.

8. The stoma plug according to any of the previous claims, wherein the tube (10) comprises a rounded tip (15) on its distal portion, configured to facilitate the insertion of the tube (10) through the stoma (45).

9. The stoma plug according to any of the previous claims, wherein the tube (10) external surface further comprises at least a spiral portion configured to increase the contact surface of the tube (10) with the inner surface of the colon, ileum and/or ureter (50).

10. The stoma plug according to any of the previous claims, wherein the tube (10) is comprised of any of the materials selected from: medical grade silicone, surgical plastic or thermoplastic elastomers.

11. The stoma plug according to any of the previous claims, wherein the tube (10) further comprises a lubricated layer on its external surface, configured to reduce the friction with the stoma (45) during its insertion.

12. The stoma plug according to any of the previous claims, wherein the two-way valve system (20) further comprises a heat-sensitive hydrocolloid surface configured to stick to the external stoma mucosa (45).

13. The stoma plug according to the previous claim, wherein the heat-sensitive hydrocolloid surface comprises a dressing configured to adhere to the subject's skin (40) extending radially from the two-way valve system (20), preferably wherein the dressing is transparent.

14. A stoma plug system comprising:
a) a stoma plug according to any of the previous claims, and
b) a collecting bag or a vacuum device
wherein the collecting bag or vacuum device is configured to connect to the two-way valve system (20) and to open the at least one opening (21) by displacing the plug (24) from the at least one opening (21) more preferably, wherein the collecting bag or vacuum device opens the at least one opening (21) through its connection to the two-way valve system (20).

15. The stoma system according to claim 14, wherein the vacuum device is a syringe.
